# EUROPÄISCHE PATENTSCHRIFT

(11) **EP 0 739 616 B1**
(45) Veröffentlichungstag und Bekanntmachung des Hinweises auf die Patenterteilung: **28.07.1999**
(21) Anmeldenummer: 95106878.2
(22) Anmeldetag: 06.05.1995
(51) Int. Cl.: A61F 5/01

(54) **Vorfuss-Entlastungsschuh, insbesondere zur postoperativen Behandlung**
Fore-foot relief shoe especially for postsurgical use
Chaussure ouverte à l'avant-pied notamment pour usage post-chirurgical

(30) Priorität: 25.04.1995 DE 29506925 U
(43) Veröffentlichungstag der Anmeldung: 30.10.1996
(73) Patentinhaber: Podia Trade Heil- und Hilfsmittel GmbH & Co. KG, 21337 Lüneburg (DE)
(72) Erfinder: Prahl, Jan, D-21379 Rullstorf (DE)
(74) Vertreter: Richter, Werdermann & Gerbaulet

(56) Entgegenhaltungen:
- EP-A- 0 248 964
- EP-A- 0 538 758
- DE-U- 9 411 782
- US-A- 3 680 550
- US-A- 4 546 557

## Beschreibung

Die Erfindung betrifft einen Vorfußentlastungsschuh,insbesondere zur postoperativen Behandlung, mit einem in einer Seitenansicht oder einem vertikalen Längsschnitt im wesentlichen dreieckigen Sohlenteil mit nach hinten abnehmender Dicke, das vor dem Mittelfußbereich des Schuhs endet.

Ein solcher Vorfußentlastungsschuh ist aus der US-A 4,546,557 bekannt. Chirurgische Eingriffe im Vorfußbereich, insbesondere bei Hallux Valgus Operationen, erfordern eine postoperative Schonung des (noch) nicht belastbaren Vorderfußes. Will der Patient während dieser Zeit nicht auf Krücken laufen, bietet sich der eingangs genannte Entlastungsschuh an, dessen schräggestelltes Fußbett in Verbindung mit dem vor dem Mittelfußbereich endenden Sohlenteil sicherstellt, daß der Patient ohne Vorfußbelastung auftreten und auch gefahrlos abrollen kann. Der Schuh nach der genannten US-Patentschrift besitzt eine kompressible Sohle mit einer von hinten nach vorn wachsenden Kompressibilität. Diese Ausgestaltung soll dazu dienen, Stöße im Vorderbereich des Schuhs besser absorbieren zu können. Abgesehen davon, daS die Einstellung einer unterschiedlichen Kompressibilität in einem Sohlenteil fertigungstechnisch schwierig und/oder aufwendig ist, sind Kunststoff-Sohlenteile bei Benutzung erheblichen mechanischen Beanspruchungen ausgesetzt, die bei Materialermüdung leicht zu einem Ausbrechen von großen Kunststoffteilen führen können, wonach das Sohlenteil unbrauchbar ist. Einer im Prinzip möglichen Armierung dieses Sohlenteils stehen die Forderungen nach einem möglichst geringen Gewicht ebenso entgegen wie der gewünschten Elastizität des Sohlenteils beim Abrollen.

Es ist daher Aufgabe der vorliegenden Erfindung, den Schuh dahingehend weiterzuentwickeln, daß er ohne Schädigung des Laufblockes längere Tragzeiten bzw. Benutzungszeiten ermöglicht und einen guten Tragkomfort sicherstellt, insbesondere hinsichtlich der gewünschten unterschiedlichen Sohlenteil-Elastizität.

Diese Aufgabe wird durch den Vorfußentlastungsschuh mit den im Anspruch 1 aufgeführten Merkmalen gelöst.

Erfindungsgemäß besteht das Sohlenteil aus einem synthetischen Schaumstoff, in den eine Feder eingebettet ist, die entgegen dem durch einen Fuß auf den Schuh ausgeübten Druck spannbar ist. Diese Feder bewirkt somit eine dauerelastische Armierung, bei der durch Einstellen der Federkonstanten in jedem Sohlenteil (Segment) die gewünschten Rückstellkräfte in unterschiedlichem Maße ebenso realisierbar sind wie durch die Feder das verwendete synthetische Schaumstoffmaterial, das ein beliebiger Kunststoff sein kann, in entsprechender Weise die Haltbarkeit des Sohlenteils verbessert. Die Erfindung kann mit nur einer einzigen Feder oder einer Gruppe von Federn realisiert werden, die unabhängig voneinander oder in Verbindung miteinander in den Schaumstoff eingebettet sind.

Vorzugsweise erstreckt sich die Feder bzw. erstrecken sich die Federn im wesentlichen über die gesamte Sohlenlänge (mit Ausnahme von Randbereichen).

Nach einer weiteren Ausgestaltung der Erfindung besitzt der Vorfußentlastungsschuh ein Sohlenteil mit einer von hinten nach vorn wachsenden Kompressibilität, die den Abrollkomfort verbessert.

In einer besonderen Ausführungsform der Erfindung ist an der Sohlenoberseite eine Blattfeder eingebettet, die vorzugsweise bündig mit den Randbereichen der Sohlenoberseite abschließt. Auf diese Weise ergibt sich eine glatte Auflagefläche, die leicht an die Entlastungsschuhfußbettsohle angelegt und befestigt werden kann und bei der die Feder ihre Wirkung fußbettsohlennah übertragen kann. Die unterschiedliche Elastizität wird im Gegensatz zum Stand der Technik nicht durch den homogenen synthetischen Sohlenteil-Kunststoffkörper, sondern durch die Feder gewährleistet.

Nach einer weiteren Verbesserung liegen an der Unterseite der Blattfeder die endseitigen abgebogenen Flachprofile einer bogenförmigen Feder an oder sie sind hiermit verbunden. Die Kombination einer oben liegenden Blattfeder mit einer bogenförmigen Feder erlaubt eine Federform-Adaption an die Sohlenteil-Geometrie in Form einer Armierung, die im wesentlichen über den gesamten Sohlenteil-Körper erstreckbar ist.

Wie im Prinzip nach dem Stand der Technik bekannt, weist das einstückige Sohlenteil eine vordere, über den im Längsquerschnitt dreieckigen Sohlenteil hinausragende Stützträgerplatte auf, an die sich S-bogenförmig die Sohlenteilvorderfläche bis hin zur Sohlenteilbodenfläche erstreckt. In diesem Falle erstreckt sich die Blattfeder bis in den Bereich der Stützträgerplatte.

Nach einer weiteren Ausgestaltung der Erfindung weist die bogenförmige Feder einen ersten vorderen Schenkel auf, der zu der unteren, sich an die Stützträgerplatte anschliessenden Sohlenteilvorderfläche etwa parallel und/oder senkrecht zur Sohlenteiloberfläche verläuft. Der zweite Schenkel der bogenförmigen Feder verläuft vorzugsweise zu der Sohlenteiloberfläche unter einem spitzen Winkel, der vorzugsweise zwischen 35° und 55° liegt. Zwischen den beiden Schenkeln liegt ein im wesentlichen kreisförmiges Mittelstück, so daß die bogenförmige Feder im Querschnitt etwa die Form eines schräggestellten V-Profils mit unterschiedlichen Neigungen der beiden Schenkel ergibt. Vorzugsweise ist das vordere abgebogene Flachprofil der bogenförmigen Feder im Übergangsbereich von der Stützträgerplatte zum dreieckigen Sohlenteil und/oder das hintere abgebogene Flachprofil kurz vor dem Fersenbeinhöcker des Fußes des Trägers anliegend angeordnet.

Um zu gewährleisten, daß sich die Feder im Kunststoffkörper nicht verschieben kann, besitzen die Blattfeder und/oder die bogenförmige Feder mindestens eine Durchbrechung, die von dem synthetischen Schaumstoff durchgriffen ist. Die Durchbrechungen können bei Flachprofil-Federn in Form von Bohrungen eingebracht sein. Nach Ausrichtung der Federn in einer Spritzgießvorrichtung wird das Sohlenteil-Kunststoffmaterial in die Spritzform, gegebenenfalls unter Druck, eingegossen, wo es unter Einschluß der Durchbrechungen die freien Spritzgießraumhohlräume durchströmt und sich nach Aushärtung vollflächig unter Durchdringung der Durchbrechungen an die Federn anlegt und aushärten kann. Vorzugsweise sind die Durchbrechungen im Querschnitt längsoval. Als Lage für die Durchbrechung bietet sich nach einer weiteren Ausgestaltung der Erfindung bei der Blattfeder der Ort vor der Anlagefläche mit der bogenförmigen Feder an und bei der bogenförmigen Feder der hintere Schenkel, der konstruktionsgemäß länger ist als der vordere Schenkel.

Vorzugsweise besteht die Feder aus einem Elastomer-Kunststoff mit einer höheren Härte als der synthetische Schaumstoff des Sohlenteils.

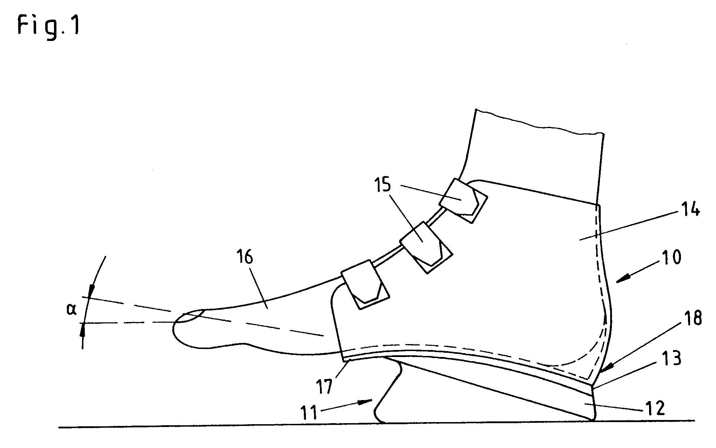

Ein Ausführungsbeispiel der Erfindung ist in den Zeichnungen dargestellt. Es zeigen
Fig. 1 eine schematische Seitenansicht eines Vorfußentlastungsschuhs und
Fig. 2 einen Längsquerschnitt durch ein Sohlenteil.

Der Vorfußentlastungsschuh 10 besteht im wesentlichen aus einem Sohlenteil 11, gegebenenfalls einem Zwischenstück oder Oberkörper 12, der Teil eines Fußbettes 13 sein kann, sowie einem aus zwei mit dem Oberkörper bzw. dem Fußbett verbundenen Schalen 14 bestehenden Teil, die beispielsweise über Klettverschlüsse 15 verschlossen werden können. Die Schalen 14 können aus Leder, Kunststoff oder Textilgewebe bestehen. Der Vorfußentlastungsschuh ist so geformt, daß der Vorfuß 16 freiliegt und zur Fußspsitze hin angehoben wird, was durch die im wesentlichen dreieckige Kontur des Sohlenteils 11 bewirkt wird. Der Neigungswinkel α liegt regelmäßig zwischen 5° und 15°. Das Fußbett 13 endet mit seinem vorderen Punkt 17 vor dem Mittelfußbereich, der nicht belastet werden soll. Beim Gehen kann der Träger des Entlastungsschuhs 10 zunächst im Bereich der Ferse 18 den Schuh aufsetzen, nach vorne hin abrollen, ohne Gefahr laufen zu müssen, mit dem Vorfuß bzw. den Zehen den Boden zu berühren.

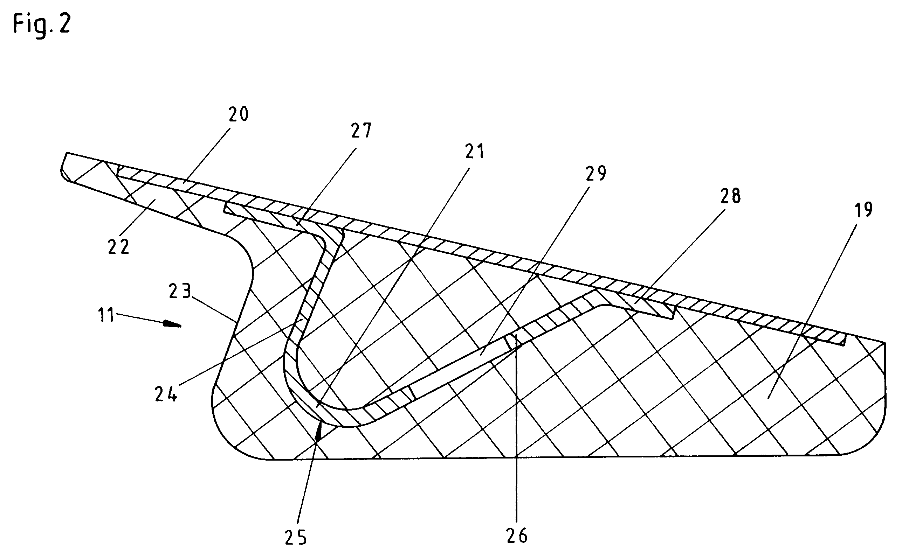

Wie die Längsquerschnitts-Ansicht des Sohlenteils 11 in Fig.2 zeigt, ist dieses als einstückiges homogenes Schaumstoffteil 19 ausgebildet, in den eine Feder eingebettet ist, die aus zwei Teilen, nämlich einer Blattfeder 20 und einer bogenförmigen Feder 21 besteht. Die Blattfeder 20 erstreckt sich bis auf einen seitlichen Randbereich vollflächig über die gesamte Länge und Breite des Sohlenteils 11, insbesondere erstreckt sie sich bis in den Bereich einer vorderen Stützträgerplatte 22 hinein, die das im übrigen dreieckförmige Sohlenteil 11 überragt und an die sich S-bogenförmig die Sohlenteilvorderfläche 23 anschließt. Die bogenförmige Feder 21 besteht aus einem vorderen Schenkel 24, einem bogenförmigen Basisteil 25 und einem hinteren Schenkel 26. An den vorderen und hinteren Schenkel schließen sich endseitig abgebogene Flachprofile 27 und 28 an, die vollflächig an der Unterseite der Blattfeder 20 anliegen. Das vordere Flachprofil 27 liegt etwa in dem Bereich der Verlängerung der Vorderwand, d.h. im Übergangsbereich zwischen der Stützträgerplatte 22 und dem im wesentlichen dreieckigen Sohlenteilkörper 19. Der vordere Schenkel ist etwa der Sohlenteilvorderfläche bzw. -wand 23 im Abstand parallel und senkrecht zur Blattfeder 20 angeordnet, wohingegen der hintere Schenkel 26 unter einem spitzen Winkel gegen die Blattfeder 20 ausläuft. Zusätzlich besitzt der hintere Schenkel 26 eine längsovale Durchbrechung 29, die vom Schaummaterial des Sohlenteils 19 durchgriffen wird. Die Blattfeder 20 kann entsprechende Durchbrechungen besitzen, vorzugsweise im Bereich der vorderen Stützträgerplatte 22 und/oder im hinteren Bereich, d.h. hinter dem endseitig abgebogenen Flachprofil 28.

Der Vorfußentlastungsschuh mit dem nach Fig.2 näher spezifizierten Sohlenteil ist gegenüber den nach dem Stand der Technik bekannten Ausführungsformen optimiert. Als Sohlenteil-Material kann eine fertigungstechnisch einfach herstellbare homogene Gummisubstanz gewählt werden, die durch die Armierung mittels der Feder 20,21 verstärkt wird, so daß diese Gummisubstanz über eine längere Zeit den Belastungen beim Gehen standhalten kann. Durch die Feder 20,21 wird eine Lastverteilung erreicht, welche die verschleißende Kompression des Schaumstoffes oder der Gummisubstanz ausschaltet. Hierdurch kann es von vornherein nicht zu Überbeanspruchungen bei starken Kompressionen kommen. Die Feder 20,21 ist so gestaltet, daß sie die gesamte Belastungsfläche der Oberseite abdeckt und in den vorderen Abrollbereich hinein mit einer rundbogenförmigen Aussteifung 24,25 ausgestattet ist. Die abgebogenen Flachprofile 27 und 28 können an der Unterseite der Blattfeder 20 durch Kleben befestigt sein. Die doppellagige Feder 20,21 verstärkt die Vorfußflexibilität und unterstützt die Abrollbewegung dergestalt, daß zur Ferse hin ein weicher Auftritt zur Abpufferung der Stöße erreicht wird. In der Abrollkante wird durch die Federn 20,21 ein erhöhter Druck aufgebracht, womit der Vorfuß freischwebend entlastet wird.

Als Material kann ein Kunststoff gewählt werden, der temperaturunabhängig mechanisch stabil ist, d.h. bei Frost ebenso bruchfrest ist wie bei extremen Wärmeeinflüssen bis zu 40°C. Gegebenenfalls wird die Feder 20,21 mittels Haftvermittler so vorbehandelt, daß sie mit dem Schaummaterial 19 eine intensive Verwachsung eingeht. Auch Kohlefasern können als Herstellungsmaterial für die Feder 20,21 verwendet werden.

## Patentansprüche

1. Vorfußentlastungsschuh (10), insbesondere zur postoperativen Behandlung, mit einem in einer Seitenansicht oder einem vertikalen Längsquerschnitt im wesentlichen dreieckigen Sohlenteil (11) mit nach hinten abnehmender Dicke, das vor dem Mittelfußbereich des Schuhs endet,
dadurch gekennzeichnet,
daß das Sohlenteil (11) aus einem homogenen synthetischen Schaumstoff oder Gummisubstanz (19) besteht, in den eine Feder (20, 21) eingebettet ist, die entgegen dem durch einen Fuß auf den Schuh ausgeübten Druck spannbar ist.

2. Vorfußentlastungsschuh nach Anspruch 1,
dadurch gekennzeichnet,
daß sich die Feder (20, 21) im wesentlichen über die gesamte Sohlenlänge erstreckt.

3. Vorfußentlastungsschu nach Anspruch 1 oder 2,
dadurch gekennzeichnet,
daß der Schuh ein Sohlenteil (11) mit einer von hinten nach vorn wachsenden Kompressibilität aufweist.

4. Vorfußentlastungsschuh nach einem der Ansprüche 1 bis 3,
dadurch gekennzeichnet,
daß an der Sohlenoberseite eine Blattfeder (20) eingebettet ist, die vorzugsweise bündig mit den Randbereichen der Sohlenoberseite abschließt.

5. Vorfußentlastungsschuh nach Anspruch 4,
dadurch gekennzeichnet,
daß an der Unterseite der Blattfeder (20) die endseitigen, abgebogenen Flachprofile (27, 28) einer bogenförmigen Feder (21) anliegen oder hiermit verbunden sind.

6. Vorfußentlastungsschuh nach Anspruch 5,
dadurch gekennzeichnet,
daß das einstückige Sohlenteil (11) eine vordere, über den im Längsquerschnitt dreieckigen Sohlenteil hinausragende Stützträgerplatte (22) aufweist.

7. Vorfußentlastungsschuh nach Anspruch 5 oder 6,
dadurch gekennzeichnet,
daß die bogenförmige Feder (21) einen ersten vorderen Schenkel (24) aufweist, der zu der unteren, sich an die Stützträgerplatte (22) anschließenden Sohlenteilvorderfläche (23) etwa parallel und senkrecht zu der Sohlenteiloberfläche verläuft und einen zweiten Schenkel (26) besitzt, der zu der Sohlenteiloberfläche unter einem spitzen Winkel verläuft, der vorzugsweise zwischen 35° und 55° liegt.

8. Vorfußentlastungsschuh nach Anspruch 5 oder 6,
dadurch gekennzeichnet,
daß die bogenförmige Feder (21) einen ersten vorderen Schenkel (24) aufweist, der zu der unteren, sich an die Stützträgerplatte (22) anschließenden Sohlenteilvorderfläche (23) etwa parallel verläuft und einen zweiten Schenke (26) besitzt, der zu der Sohlenteiloberfläche unter einem spitzen Winkel verläuft, der vorzugsweise zwischen 35° und 55° liegt.

9. Vorfußentlastungsschuh nach Anspruch 5 oder 6,
dadurch gekennzeichnet,
daß die bogenförmige Feder (21) einen ersten vorderen Schenkel (24) aufweist, der zu der unteren, sich an die Stützträgerplatte (22) anschließenden Sohlenteilvorderfläche (23) senkrecht zu der Sohlenteiloberfläche verläuft und einen zweiten Schenkel (26) besitz, der zu der Sohlenteiloberfläche unter einem spitzen Winkel verläuft, der vorzugsweise zwischen 35° und 55° liegt.

10. Vorfußentlastungsschuh nach einem der Ansprüche 5 bis 9,
dadurch gekennzeichnet,
daß das vordere abgebogene Flachprofil (27) der bogenförmigen Feder (21) im Übergangsbereich von der Stützträgerplatte (22) zum dreieckigen Sohlenteil (11) liegt und das hintere abgebogene Flachprofil (28) der bogenförmigen Feder (21) vor dem Ende des Sohlenteils (11) endet.

11. Vorfußentlastungsschuh nach einem der Ansprüche 5 bis 9,
dadurch gekennzeichnet,
daß das vordere abgebogene Flachprofil (27) der bogenförmigen Feder (21) im Übergangsbereich von der Stützträgerplatte (22) zum dreieckigen Sohlenteil (11) liegt.

12. Vorfußentlastungsschuh nach einem der Ansprüche 5 bis 9,
dadurch gekennzeichnet,
daß das hintere abgebogene Flachprofil (28) der bogenförmigen Feder (21) vor dem Ende des Sohlenteils (11) endet.

13. Vorfußentlastungsschuh nach einem der Ansprüche 1 bis 12,
dadurch gekennzeichnet,
daß die Blattfeder (20) und die bogenförmige Feder (21) ein Flachprofil mit mindestens einer Durchbrechung (29) ist, die von dem synthetischen Schaumstoff oder Gummisubstanz (19) durchgriffen ist.

14. Vorfußentlastungsschuh nach einem der Ansprüche 1 bis 12,
dadurch gekennzeichnet,
daß die Blattfeder (20) ein Flachprofil mit mindestens einer Durchbrechung (29) ist, die von dem synthetischen Schaumstoff oder Gummisubstanz (19) durchgriffen wird.

15. Vorfußentlastungsschuh nach einem der Ansprüche 1 bis 12,
dadurch gekennzeichnet,
daß die bogenförmige Feder (21) ein Flachprofil mit mindestens einer Durchbrechung (29) ist, die von dem synthetischen Schaumstoff oder Gummisubstanz (19) durchgriffen wird.

16. Vorfußentlastungsschuh nach einem der Ansprüche 13 bis 15,
dadurch gekennzeichnet,
daß die Durchbrechung (29) im Querschnitt längsoval ist.

17. Vorfußentlastungsschuh nach einem der Ansprüche 13 bis 16,
dadurch gekennzeichnet,
daß die Blattfeder (20) eine Durchbrechung aufweist, die im Bereich der vorderen Stützträgerplatte (22) und/oder im hinteren Bereich hinter dem endseitig abgebogenen Flachprofil (28) liegt.

18. Vorfußentlastungsschuh nach einem der Ansprüche 1 bis 17,
dadurch gekennzeichnet,
daß die bogenförmige Feder (21) im hinteren Schenkel eine Durchbrechung (29) aufweist.

19. Vorfußentlastungsschuh nach einem der Ansprüche 1 bis 18,
dadurch gekennzeichnet, daß die Feder (20, 21) aus einem Elastomer-Kunststoff besteht.

## Claims

1. Forefoot relief shoe (10), especially for post-surgical use, with a sole portion (11) which is essentially triangular in a side view or in a longitudinal cross section, the thickness of which decreases towards the rear, which terminates before the metatarsal region of the shoe,
characterized in that
the sole portion (11) is comprised of a homogeneous synthetic foamed material or of a rubber substance (19), wherein a spring (20,21) is embedded that can be tensed against a pressure exerted by a foot on the shoe.

2. Forefoot relief shoe according to Claim 1,
characterized in that
the spring (20,21) extends essentially across the entire length of the shoe.

3. Forefoot relief shoe according to either Claim 1 or 2,
characterized in that
the shoe possesses a sole portion (11) with a compressibility increasing from the rear to the front.

4. Forefoot relief shoe according to any of Claims 1 to 3,
characterized in that,
on the top side of the sole, a leaf spring (20) is embedded, which preferably terminates flush with the marginal areas of the sole top side.

5. Forefoot relief shoe according to Claim 4,
characterized in that
the terminal bent-aside flat sections (27,28) of an arcuate spring (21) rest against the underside of the leaf spring (20) or are connected hereto.

6. Forefoot relief shoe according to Claim 5,
characterized in that
the single-piece sole portion (11) possesses an anterior supporting plate (22) proceting over the, in the longitudinal cross section, triangular sole portion.

7. Forefoot relief shoes according to either Claim 5 or 6,
characterized in that
the arcuate spring (21) possesses a first front leg (24), which, relative to the lower sole portion front surface (23) following the the supporting plate (22), proceeds approximately parallel and vertically to the sole portion top surface and possesses a second leg (26), which, in relation to the sole portion surface, proceeds at an acute angle which preferably ranges between 35° and 55°.

8. Forefoot relief shoe according to either Claim 5 or 6,
characterized in that
the arcuate spring (21) possesses a first leg (24), which, relative to the lower sole portion front surface (23) following the supporting plate (22), proceeds approximately parallel and possesses a second leg (26), which, relative to the sole portion surface, proceeds at an acute angle preferably ranging between 35° and 55°.

9. Forefoot relief shoes according to either Claim 5 or 6,
characterized in that
the arcuate spring (21) possesses a first front leg (24), which proceeds, relative to the lower sole portion front surface (23) following the supporting plate (22), vertically to the sole portion front surface and possesses a second leg (26), which, in relation to the sole portion surface, which, relative to the sole portion surface, proceeds at an acute angle which ranges preferably between 35° and 55°.

10. Forefoot relief shoes according to any of Claims 5 to 9,
characterized in that
the anterior bent-aside flat section (27) of the arcuate spring (21) is located within the transitional region from the supporting plate (22) to the triangular sole portion (11) and in that the posterior bent-aside flat section (28) of the arcuate spring (21) terminates before the extremity of the sole portion (11).

11. Forefoot relief shoe according to any of Claims 5 to 9,
characterized in that
the anterior bent-aside flat section (27) of the arcuate spring (21) is located within the transitional region from the supporting plate (2) to the triangular sole portion (11).

12. Forefoot relief shoe according to any of Claims 5 to 9,
characterized in that
the posterior bent-aside flat section (28) of the arcuate spring (21) terminates before the extremity of the sole portion (11).

13. Forefoot relief shoe according to any of Claims 1 to 12,
characterized in that
the leaf spring (20) and the arcuate spring (21) is a flat section with at least one perforation (29), which is reached through by the synthetic foamed material or by the rubber substance (19).

14. Forefoot relief shoe according to any of Claims 1 to 12,
characterized in that
the leaf spring (20) is a flat section with at least one perforation (29), which is reached through by the synthetic foamed material or by the rubber substance (19).

15. Forefoot relief shoe according to any of Claims 1 to 12,
characterized in that
the arcuate spring (21) is a flat section with at least one perforation (29), which is passed through by the synthetic foamed material or by the rubber substance (19).

16. Forefoot relief shoe according to any of Claims 13 to 15,
characterized in that
the perforation (29) is longitudinally oval in cross section.

17. Forefoot relief shoe according to any of Claims 13 to 16,
characterized in that
the leaf spring (20) possesses a perforation which is located within the region of the anterior suporting plate (22) and/or within the posterior region behind the flsat section (28) bent aside at the extremity.

18. Forefoot relief shoe according to any of Claims 1 to 17,
characterized in that
the arcuate spring (21) possesses a perforation (29) in the rear leg.

19. Forefoot relief shoe according to any of Claims 1 to 18,
characterized in that
the spring (20,21) is comprised of an elastomer plastic.

## Revendications

1. Chaussure ouverte à l'avant-pied (10), notamment pour usage post-chirurgical, avec une partie semelle (11), substantiellement triangulaire dans une vue latérale ou dans une section longitudinale verticale, avec une épaisseur qui diminue vers l'arrière, qui se termine deant la zone médiane du pied de la chaussure,
caractérisée en ce
que la partie semelle (11) est constituée par une matière alvéolaire synthétique homogène ou par une substance de caoutchouc (19) dans laquelle est encastré un ressort (20, 21) qui peut être tendu à l'encontre de la pression exercée par un pied sur la chaussure.

2. Chaussure ouverte à l'avant-pied selon la revendication 1,
caractérisée en ce
que le ressort (20, 21) s'étend substantiellement sur toute la longueur de la semelle.

3. Chaussure ouverte à l'avant-pied selon la revendication 1 ou 2,
caractérisée en ce
que la chaussure présente une partie semelle (11) avec une compressibilité croissante de l'arrière à l'avant.

4. Chaussure ouverte à l'avant-pied selon l'une des revendications 1 à 3,
caractérisée en ce
qu'un ressort à lames (20) est encastré sur la face supérieure de la semelle, ressort qui se termine de préférence à fleur avec les zones des bords de la face supérieure de la semelle.

5. Chaussure ouverte à l'avant-pied selon la revendication 4,
caractérisée en ce
que les profilés plats (27, 28) recourbés d'extrémité d'un ressort en forme d'arc (21) reposent sur la face inférieure du ressort à lames (20) ou sont reliés à celle-ci.

6. Chaussure ouverte à l'avant-pied selon la revendication 5,
caractérisée en ce
que la partie de semelle en une pièce (11) présente une plaque de support d'appui (22) antérieure qui fait saillie sur la partie de semelle de section longitudinale triangulaire.

7. Chaussure ouverte à l'avant-pied selon la revendication 5 ou 6,
caractérisée en ce
que le ressort en forme d'arc (21) présente un premier montant antérieur (24) qui est à peu près parallèle à la surface antérieure inférieure de la partie semelle (23) qui suit la plaque de support d'appui (22) et est à peu près perpendiculaire à la surface supérieure de la partie semelle et possède un second montant (26) qui forme un angle aigu avec la surface supérieure de la partie semelle qui se situe de préférence entre 35° et 55°.

8. Chaussure ouverte à l'avant-pied selon la revendication 5 ou 6,
caractérisée en ce
que le ressort en forme d'arc (21) présente un premier montant antérieur (24) qui est à peu près parallèle à la surface antérieure inférieure de la partie semelle (23) qui suit la plaque de support d'appui (22) et possède un second montant (26) qui forme un angle aigu avec la surface supérieure de la partie semelle qui se situe de préférence entre 35° et 55°.

9. Chaussure ouverte à l'avant-pied selon la revendication 5 ou 6,
caractérisée en ce
que le ressort en forme d'arc (21) présente un premier montant antérieur (24) qui est perpendiculaire à la surface antérieure inférieure de la partie semelle (23) qui suit la plaque de support d'appui (22) et possède un second montant (26) qui forme un angle aigu avec la surface supérieure de la partie semelle qui se situe de préférence entre 35° et 55°.

10. Chaussure ouverte à l'avant-pied selon l'une des revendications 5 à 9,
caractérisée en ce
que le profilé plat recourbé antérieur (27) du ressort en forme d'arc (21) se trouve dans la zone de transition de la plaque de support d'appui (22) et de la partie de semelle triangulaire (11) et le profilé plat recourbé postérieur (28) du ressort en forme d'arc (21) se termine devant l'extrémité de la partie semelle (11).

11. Chaussure ouverte à l'avant-pied selon l'une des revendications 5 à 9,
caractérisée en ce
que le profilé plat recourbé antérieur (27) du ressort en forme d'arc (21) se trouve dans la zone de transition de la plaque de support d'appui (22) et de la partie de semelle triangulaire (11).

12. Chaussure ouverte à l'avant-pied selon l'une des revendications 5 à 9,
caractérisée en ce
que le profilé plat recourbé postérieur (28) du ressort en forme d'arc (21) se termine devant l'extrémité de la partie semelle (11).

13. Chaussure ouverte à l'avant-pied selon l'une des revendications 1 à 12,
caractérisée en ce
que le ressort à lames (20) et le ressort en forme d'arc (21) est un profilé plat avec au moins une découpure (29) qui est traversée par la matière alvéolaire synthétique ou la substance de caoutchouc (19).

14. Chaussure ouverte à l'avant-pied selon l'une des revendications 1 à 12,
caractérisée en ce
que le ressort à lames (20) est un profilé plat avec au moins une découpure (29) qui est traversée par la matière alvéolaire synthétique ou la substance de caoutchouc (19).

15. Chaussure ouverte à l'avant-pied selon l'une des revendications 1 à 12,
caractérisée en ce
que le ressort en forme d'arc (21) est un profilé plat avec au moins une découpure (29) qui est traversée par la matière alvéolaire synthétique ou la substance de caoutchouc (19).

16. Chaussure ouverte à l'avant-pied selon l'une des revendications 13 à 15,
caractérisée en ce
que la découpure (29) est de section ovale allongée.

17. Chaussure ouverte à l'avant-pied selon l'une des revendications 13 à 16,
caractérisée en ce
que le ressort à lames (20) présente une découpure qui est située dans la zone de la plaque de support d'appui antérieure (22) et/ou dans la zone postérieure derrière le profilé plat recourbé à l'extrémité (28).

18. Chaussure ouverte à l'avant-pied selon l'une des revendications 1 à 17,
caractérisée en ce
que le ressort en forme d'arc (21) présente une découpure (29) dans le montant postérieur.

19. Chaussure ouverte à l'avant-pied selon l'une des revendications 1 à 18,
caractérisée en ce
que le ressort (20, 21) est constitué par une matière synthétique à base d'élastomère.
